# EUROPEAN PATENT SPECIFICATION

(11) **EP 2 451 387 B1**
(45) Date of publication and mention of the grant of the patent: **01.07.2015**
(21) Application number: 10797373.7
(22) Date of filing: 12.07.2010
(51) Int. Cl.: A61F 2/36

(54) **HIP JOINT DEVICE**
HÜFTGELENKVORRICHTUNG
PROTHÈSE DE HANCHE

(30) Priority: 10.07.2009 SE 0900981; 10.07.2009 SE 0900957; 10.07.2009 SE 0900958; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 30.07.2009 US 229755 P; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, CH-6300 Zug (CH)
(86) International application number: PCT/SE2010/000192
(87) International publication number: WO 2011/005166

(56) References cited:
- WO-A1-2008/104072
- WO-A1-2008/104072
- DE-A1-102007 018 341
- DE-U1- 20 003 360
- DE-U1- 20 003 360
- FR-A- 1 061 009
- FR-A1- 2 357 229
- FR-A1- 2 578 739
- US-A- 3 053 251
- US-A- 4 123 806
- US-A1- 2004 059 429
- US-B2- 7 241 315
- US-B2- 7 241 315

## Description

### Technical field

The present invention relates generally to medical devices for implantation in a hip joint.

### Background art

The hip joint is a synovial joint, joining the pelvis to the proximal portion of the femoral bone. Synovial joints are the most common types of joints in mammals, and are typical of nearly all limb joints. The contacting surfaces of said the pelvic, the acetabulum, and the contacting surface of the femoral bone, the caput femur, are smooth and rounded, and covered by articular cartilage. A synovial membrane, encapsulates the joint, forming a hip joint cavity, which contains synovial fluid. Outside the synovial membrane is a fibrous capsule and ligaments, forming an articular capsule.

There are both natural and pathological processes leading to deteriorated joint function. With age and wear, the articular cartilage becomes less effective as a shock absorber and a lubricated surface. Different degenerative joint diseases, such as arthritis, osteoartrithis, or osteoarthrosis, accelerate the deterioration.

Hip joint Osteoarthritis is a syndrome in which low-grade inflammation results in pain in the hip joints, caused by abnormal wearing of the Cartilage that acts as a cushion inside if the hip joint. This abnormal wearing of the cartilage also results in a decrease of the joints lubricating fluid called Synovial fluid. Hip joint Osteoarthritis is estimated to affect 80% of all people over 65 years of age, in more or less serious forms.

The present treatment for hip osteoarthritis comprises NSAID drugs, local injections of Hyaluronic acid or Glucocorticoid to help lubricating the hip joint, and replacing parts of the hip joint with a prosthesis through hip joint surgery.

The replacing of parts of the hip joint is one of the most common surgeries to date performed at hundreds of thousands of patients in the world every year. The most common method comprises placing a metal prosthesis in Femur and a plastic bowl in Acetabulum. This operation is done through an incision in the hip and upper thigh and through Fascia Lata and the lateral muscles of the thigh. To get access to the joint, the supporting Capsule attached to Femur and Ilium needs to be penetrated, making it difficult to get a fully functional joint after the surgery. Femur is then cut at the neck with a bone saw and the prosthesis is placed in femur either with bone cement or without. Acetabulum is slightly enlarged using an Acetabular reamer, and the plastic bowl is positioned using screws or bone cement.

The complications after hip joint surgery includes dislocation of the hip joint and loosening of the prosthesis from its fixation in the femoral bone. The loosening and/or dislocation of the prosthesis could be induced by an abnormal strain being placed on the hip joint from e.g. the patient falling or making a rapid movement of the hip, or by a bodily macrophage reaction.
FR 2357229 (to Partridge) discloses a band for use when mending the femoral bone in connection with the implantation of a prosthetic joint.
WO 2008(104072 (to Schwartz et al.) discloses a prosthesis for use as a surface in joint surface, preferably in the temporomandibular joint.
US 3053251 (to Black et al.) discloses a prosthetic surface for use in the hip joint of a patient. DE 200 03 360 (to König) discloses a prosthetic surface for use in the hip joint of a patient.

### Summary

A device including a locking member for implantation in a hip joint of a patient is provided. The locking member is adapted to fixate a medical device comprising an artificial hollow caput femur surface to a caput femur hip joint surface. The locking member comprises a loop-shaped element with a first and a second end adapted be mechanically connected using an engagement member, so that it forms a closed loop-shaped element with a circumference encircling the caput femur or collum femur.

According to the invention, the loop-shaped element is adapted to be connected in at least a first and second predefined locking position. The loop-shaped element could have a first inner circumference, when the loop-shaped element is connected in the first locking position, the loop shaped element has a second smaller inner circumference, when the loop shaped element is connected in the second locking position. This way the locking member could be adapted for the particular patient.

According to another embodiment the loop-shaped element is further adapted to be connected in a third locking position. The loop-shaped element has a third inner circumference, when connected in the third locking position, being smaller than the first and second inner circumference.

According to yet another embodiment, the locking member could further be adapted to be arranged in an area extending a distance beyond the maximum diameter of the caput femur.

The engagement member could according to one embodiment comprise a first and a second engagement member part arranged at said first and second locking member end, respectively.

The first and second engagement member could be adapted to mechanically self connect by introducing the first engagement member into the second engagement member. According to one embodiment the first and second engagement member parts could have the shape of protrusions extending from the first and second locking member ends. The first and second engagement member could extend axially from the first and second locking member ends to form a horizontally arranged gripping claw.

According to yet another embodiment, the first and second engagement member extends radially from the first and second locking member ends, to form a vertically arranged gripping claw.

The locking member, according to any of the embodiments, could comprises a first engagement member part having the shape of a protrusion extending from the first locking member end and said second engagement member part has the form of at least one recess or hole.

According to yet another embodiment the first and second ends of the locking member could be connected by using an engagement member comprising two pivotable locking parts.

According to yet another embodiment, the first locking part could be pivotably attached both to the first end of the locking member and to the second locking part.

The second locking part could, according to one embodiment be attached to the first locking part in an engagement point arranged between the outer ends of the first locking part.

According to yet another embodiment the second locking part could be attached to the first locking part in a point arranged substantially in the middle of said the locking part.

According to yet another embodiment, the second locking part could be adapted to engage with a protruding part arranged in the second end of the locking member.

The first and second locking member ends could be adapted to be pulled together by pivoting the first locking part around its engagement point in the first end of the locking member.

According to yet another embodiment, the first and second locking member ends could be arranged overlapping each other when locked together.

According to yet another embodiment the first and second locking member ends are arranged end to end when locked together.

### Brief description of drawings

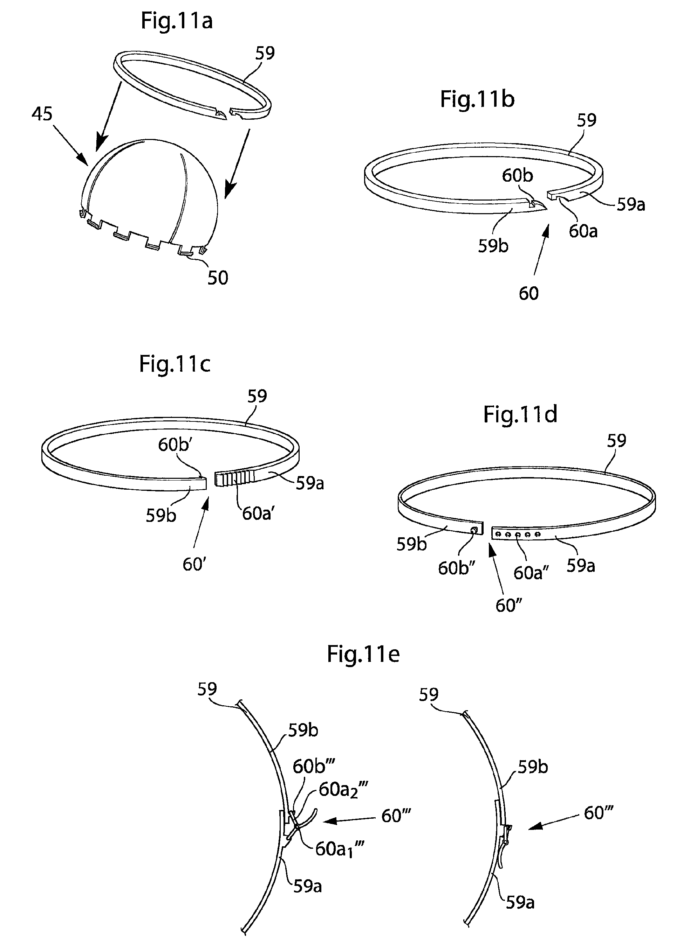

The invention is now described, by way of example, with reference to the accompanying drawings, in which only Fig. 11c and 11d show the invention as claimed.
Fig. 1 shows the hip joint of a human patient in section,
Fig. 2 shows the pelvis in a frontal view,
Fig. 3 shows the placing of an artificial caput femur surface on the caput femur in conventional surgery,
Fig 4 shows a medical device for implantation in a hip joint of a patient,
Fig. 5 shows the medical device according to one embodiment,
Fig. 6 shows the medical device according to another embodiment,
Fig. 7 shows an artificial caput femur surface in section having a major opening adapted to travel over and beyond the maximum diameter of the caput femur,
Fig. 8a shows an artificial caput femur surface 45 according to a first embodiment,
Fig. 8b shows the artificial caput femur surface when fixated to the caput femur,
Fig. 8c shows the hip joint is section when a medical device is implanted,
Fig. 9 shows an artificial acetabulum surface according to a first embodiment,
Fig. 10a-c shows an artificial acetabulum surface according to a second embodiment,
Fig. 11 a discloses the adjustable locking member to be mounted on the artificial acetabulum surface,
Fig. 11 b-11 e shows different embodiments of a locking member and an engagement member,

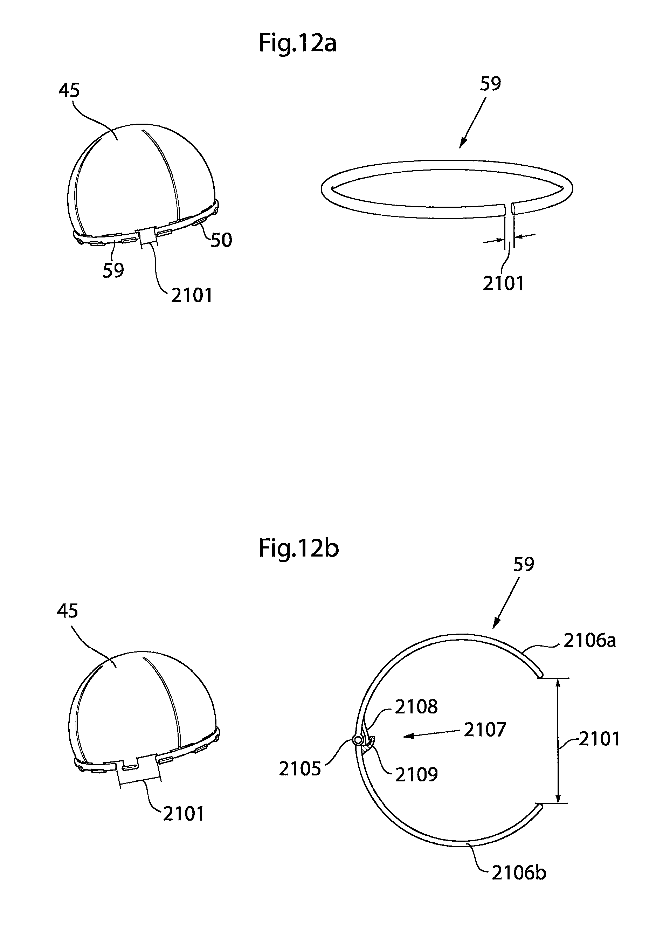

Fig. 12a shows a medical device and a locking member according to yet another embodiment,
Fig. 12b shows a medical device and a locking member according to yet another embodiment,

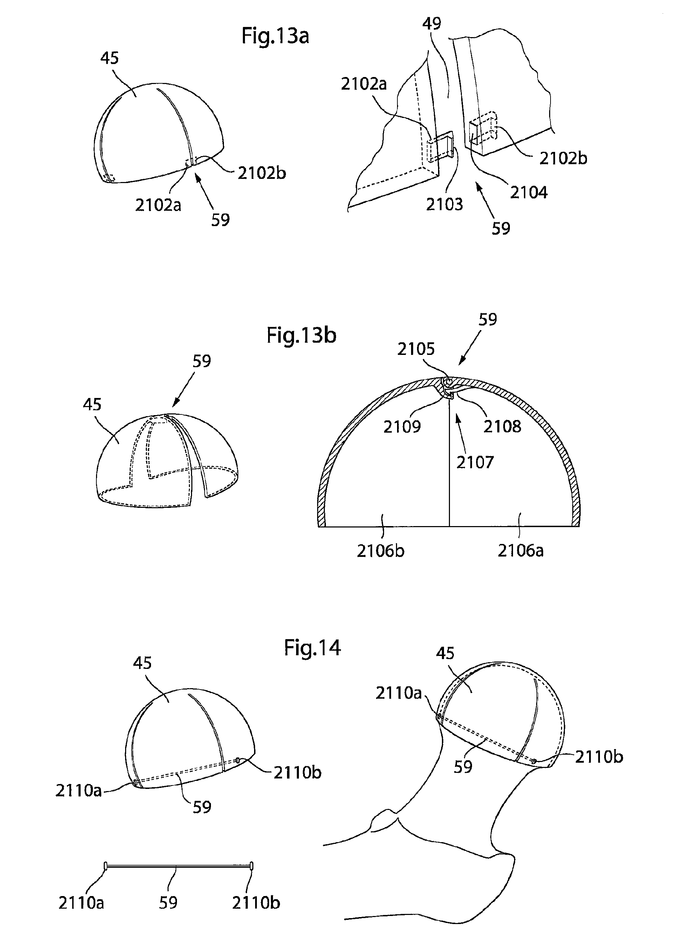

Fig. 13a shows a medical device with an integrated locking member according to one embodiment,
Fig. 13b shows a medical device with an integrated locking member according to another embodiment,
Fig. 14 shows an embodiment of a medical device and a mechanical fixating member,

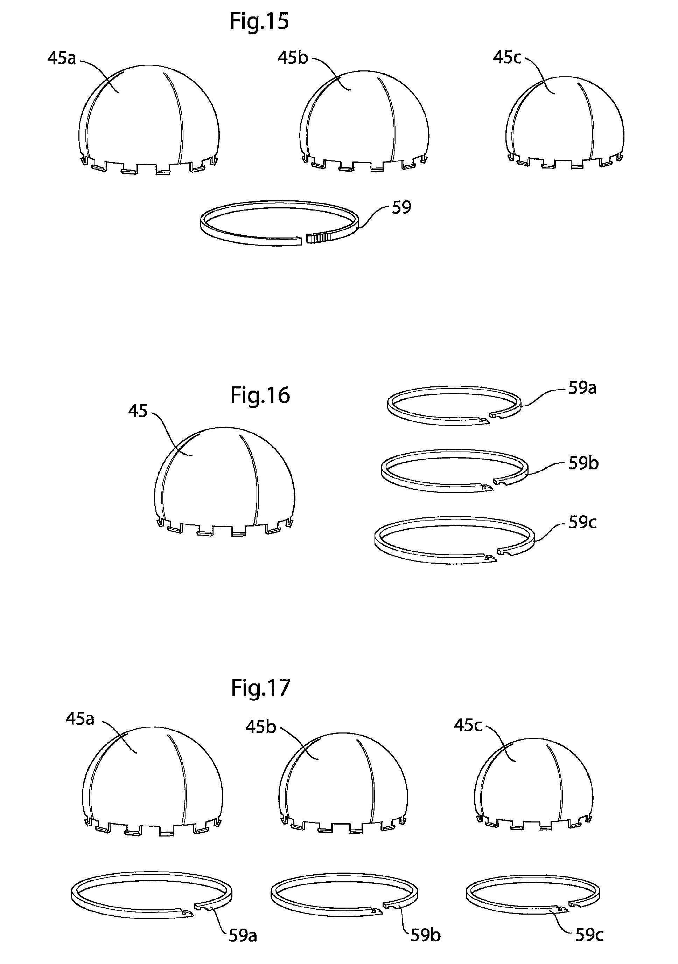

Fig. 15 shows a first kit comprising three artificial caput femur surfaces and one locking member,
Fig. 16 shows a second kit comprising one artificial caput femur surfaces and three locking members,
Fig. 17 shows a third kit comprising three artificial caput femur surfaces and three locking members.

### Detailed Description

The hip joint is a synovial ball and socket joint which permits a large motion range for allowing a plurality of different movements of the lower limb. From a neutral position the following movements of the hip joint are normally possible: Lateral or external rotation, 30° with the hip extended, 50° with the hip flexed, medial or internal rotation 40°, extension or retroversion 20°, flexion or anteversion 140°, abduction 50° with hip extended, 80° with hip flexed, adduction 30° with hip extended, 20° with hip flexed.

When replacing the natural hip joint with an artificial, the depth of the artificial acetabulum will affect the motion range of the hip joint, the deeper the acetabulum bowl is made the more restrictive it is to the motion range. A deeper bowl has the advantage of reducing the risk of hip joint luxation, the risk of which is a major drawback with prosthetic hips of today.

The caput and collum femur are to be understood as the proximal portion of the femoral bone. In orthopedic surgery the caput and/or collum femur is sometimes surgically modified, e.g. bone is removed to adapt the proximal portion of the femoral bone to a particular prosthesis. For the purpose of this application the caput/collum femur are to be understood as either the natural caput/collum femur or a surgically modified caput/collum femur.

The anatomy of the hip joint and its surroundings is further disclosed in: Marieb et al., Human Anatomy, 2003, Benjamin Cummings, San Francisco, pages 195 - 202 and in Moore et al., Clinically oriented anatomy, 1999, Lippincott, Williams & Wilkins, Baltimore, pages 501 - 653.

Centrally in the body should herein be understood as a point of reference located at the intersection of the Median plane and the Coronal plane and in the center part of the heart along a longitudinal axis (Caudal - Cranial). Proximal and distal are direction or location terms used in relation to said point centrally in the body and hence a distal point is a point farther away from the central point in relation a proximal point of the same structure. Other anatomical terms used herein are further described in Moore et al., Clinically oriented anatomy, 1999, Lippincott, Williams & Wilkins, Baltimore, pages 2 - 10, which is hereby incorporated by reference.

Functional hip movements are to be understood as movements of the hip that at least partly correspond to the natural movements of the hip. On some occasions the natural movements of the hip joint might be somewhat limited or altered after hip joint surgery, which makes the functional hip movements of a hip joint with artificial surfaces somewhat different than the functional hip movements of a natural hip joint.

Everyday activities is to be understood as activities which are not connected to any extreme movements, such that some physical sports require. For example, everyday activities comprise: walking, sitting, cycling etc.

The functional position of an implantable medical device or prosthesis is the position in which the hip joint can perform functional hip movements. The final position is to be understood as a functional position in which the medical device needs no further position change.

Elasticity is to be understood as a materials ability to deform in an elastic way.

Elastic deformation is when a material deforms under stress (e.g. external forces), but returns to its original shape when the stress is removed. A more elastic material is to be understood as a material having a lower modulus of elasticity. The elastic modulus of an object is defined as the slope of its stress-strain curve in the elastic deformation region. The elastic modulus is calculated as stress / strain, where stress is the force causing the deformation, divided by the area to which the force is applied; and strain is the ratio of the change caused by the stress.

Stiffness is to be understood as the resistance of an elastic body to deformation by an applied force.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

Form fitting is to be understood as an element having a part or section which is adapted to enable a mechanical connection of said element to at least one other element using said part or section. Form fitted structure is a structure of an element which enables form fitting.

In the following a detailed description of embodiments of the present invention will be given. In the drawing figures, like reference numerals designate identical or corresponding elements throughout the several figures. It will be appreciated that these figures are for illustration only and are not in any way restricting the scope of the invention. Thus, any references to direction, such as "up" or "down", are only referring to the directions shown in the figures. Also, any dimensions etc. shown in the figures are for illustration purposes.

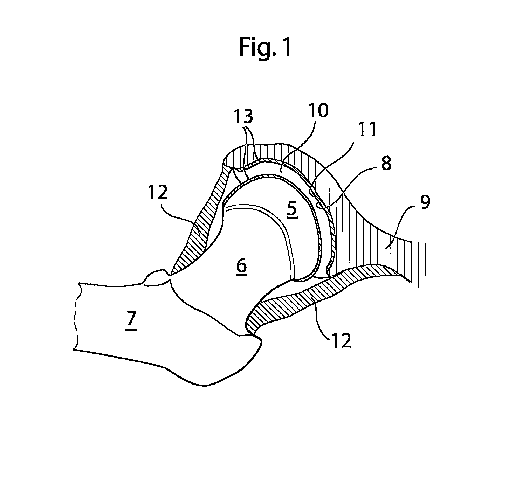

Fig. 1 shows the hip joint of a human patient in section. The hip joint comprises a caput femur 5 placed at the very top of collum femur 6 which is the top part of the femoral bone 7. The caput femur is in connection with the acetabulum 8 which is a bowl shaped part of the pelvic bone 9. Both the caput femur surface 10 and the acetabulum surface 11 are covered with articular cartilage 13 which acts as a cushion in the hip joint. In patients with hip joint osteoarthritis, this articular cartilage 13 is abnormally worn down due to a low grade inflammation. The hip joint is surrounded by the hip joint capsule 12 which provides support for the joint and hinders luxation. After conventional hip joint surgery, penetrating the hip joint capsule 12, the capsule 12 is dramatically weakened due to the limited healing possibilities of its ligament tissue. By performing hip joint surgery without damaging the hip joint capsule 12 the patient can fully recover and place equal amount of strain on an artificial joint as is possible on a natural one.

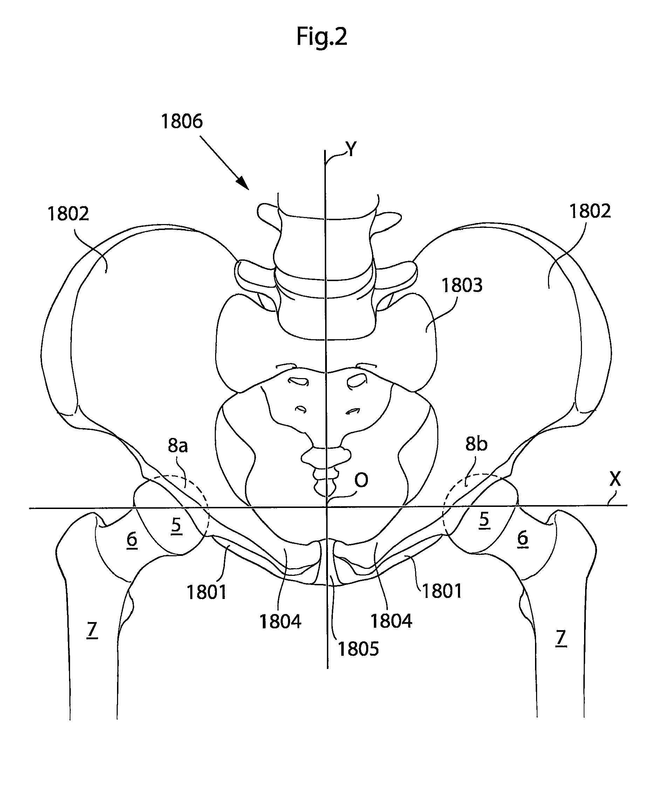

Fig. 2 shows the pelvis in a frontal view. Pelvis comprises the right and left hip bone making up the pelvic bone, in turn comprising the Sacrum 1803, Ilium 1802, Pubis 1804 and Ischium 1801. The hip joint houses the right and left acetabulum 8a,b placed laterally and distally in the pelvis. The acetabulum 8a,b being a spherically shaped cavity in the hip bones making up one of the parts of the hip joint, the acetabulum 8a,b being adapted to house the caput femur 5, being the proximal portion of the femoral bone 7 having a spherical contacting surface adapted to be placed in the acetabulum 8a,b and thus creating the operable hip joint. The pelvis has a lateral-medial axis X extending substantially from the bottom of the left acetabulum 8a to the bottom of the right acetabulum 8b, the pelvis further having a proximal-distal Y axis extending perpendicular to said lateral-medial axis, centrally and substantially along the length of the patient, passing the dorsal portions of the pubic symphysis 1805 and substantially following the spinal cord 1806, intersecting the lateral-medial axis X.

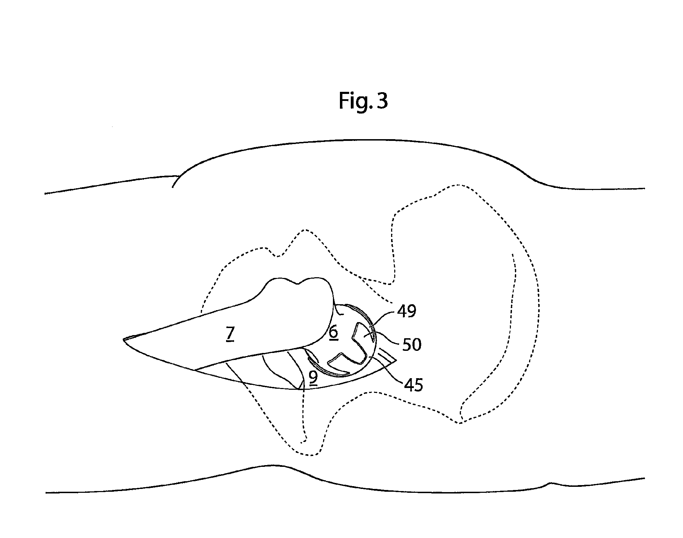

Fig. 3 shows the placing of an artificial caput femur surface 45 on the caput femur in conventional surgery. The artificial caput femur according to this embodiment comprises slits 49 and arms 50 making the structure of the artificial caput femur surface flexible for clasping the caput femur 5 and going beyond the maximum diameter of the caput femur 5. Furthermore the artificial caput femur surface 45 can be inserted into a hip joint through a hole smaller than the full functional size of the artificial caput femur surface 45, enabling a less invasive surgical procedure.

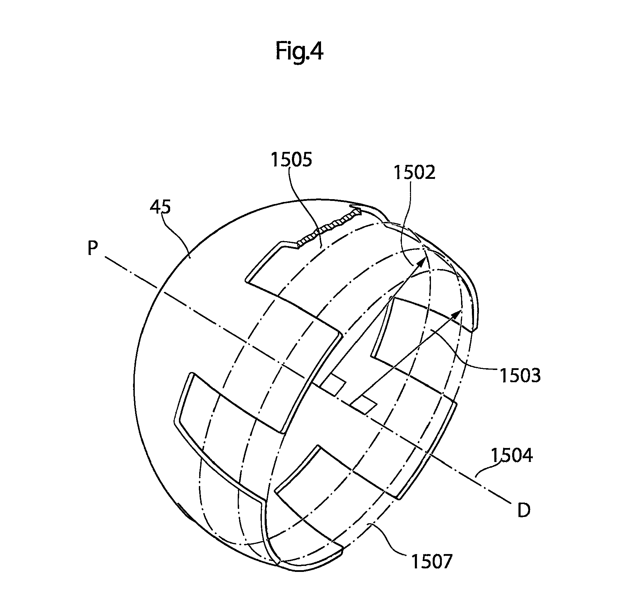

Fig 4 shows a medical device for implantation in a hip joint of a patient. The medical device comprises an artificial caput femur surface 45 being hollow, having a major opening 1507 adapted to be directed towards the caput femur or a surgically modified caput femur when implanted in the hip joint. A medical device caput center axis 1504 passes through the major opening 1507, when said medical device is implanted in a functional position in the hip joint. The medical device comprises an inner surface adapted to have a first distal distance 1503 extending perpendicularly from said medical device caput center axis 1504 to said inner surface of said artificial caput femur surface 45. The first distal distance 1503 being shorter than a second proximal distance 1502 extending perpendicularly from said medical device caput center axis 1504 to said inner surface of said artificial caput femur surface 45. The second proximal distance 1502 extending from a more proximal position on the medical device caput center axis 1504 than the first distal distance 1503, when the artificial caput femur surface 45 is implanted in a functional position in the hip joint. The maximum first distal distance 1503 is located on a part of said medical device caput center axis 1504 substantially located more proximal than said major opening 1507, when said artificial caput femur surface 45 is mounted in the functional position in the hip joint. The artificial caput femur surface 45 is adapted to have a closest second proximal 1502 distance extending perpendicularly from said medical device caput center axis 1504 to said inner surface of said artificial caput femur surface 45, being smaller than the maximum first distal distance 1503. The maximum first distal distance 1503 is extending from the medical device caput center axis 1504 more distal than the closest second proximal distance 1502, when implanted in a functional position in the hip joint.

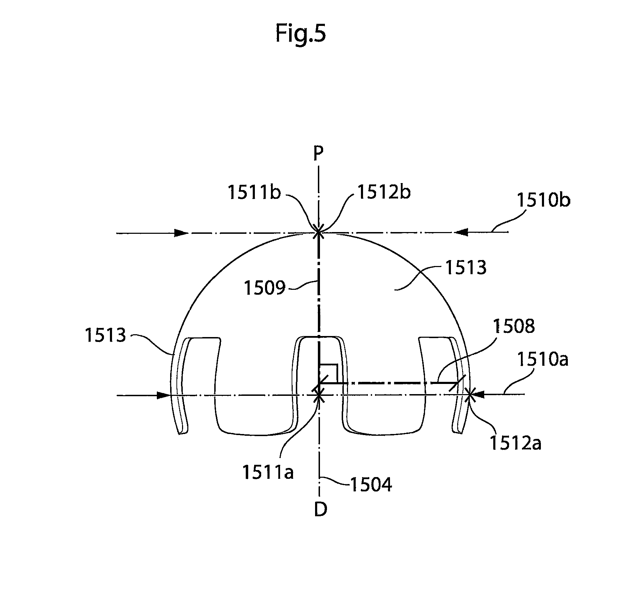

Fig. 5 shows the medical device according to one embodiment in which a maximum inner distance 1508 is extending from one or more predefined points selected along a lengthwise extending part 1509 of the medical device caput center axis 1504, in which said lengthwise extending part 1509 is defined by two cross-sections 1510a, 1510b of said artificial caput femur surface 45, extending perpendicular to the medical device caput center axis 1504, from both the distal 1511a and proximal 1511b end points of said part 1509 of the medical device caput center axis 1504, in which both cross-sections 1510a, 1510b are placed at the outer limit 1512a, 1512b of the weight carrying surface 1513 of the artificial caput femur surface, in proximal P and distal D direction, respectively. The part 1509 of the medical device caput center axis 1504 is placed between the cross-sections 1510a, 1510b.

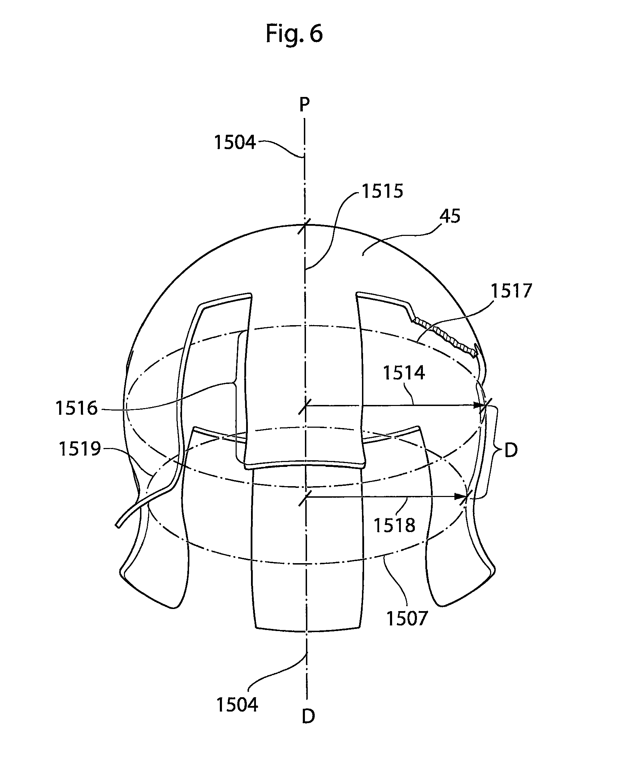

Fig. 6 shows the medical device according to an embodiment where the medical device has a maximum outer distance 1514 extending perpendicularly from a medical device caput center axis 1514 to the outer surface of the artificial caput femur surface. The artificial caput femur surface 45 comprises at least one first beyond part 1516 extending distally D, at least partly beyond a circular line 1517 of the maximum outer distance on the artificial caput femur surface 45, when implanted in said hip joint. The at least one first beyond part 1516 is adapted to have a closest distance 1518 from the inner surface of the first beyond part 1518 to the medical device caput center axis 1504, perpendicular to the medical device caput center axis 1504, being smaller than a maximum inner distance 1514 from the inner surface of the artificial caput femur surface 45 to the medical device caput center axis 1504, substantially perpendicular to the medical device caput center axis 1504. A maximum inner distance 1514 is extending from the medical device caput center axis 1504 substantially more proximal P than a major opening 1507. A lengthwise extending part of the medical device caput center axis 1504 is placed more proximal P than the major opening 1507, when the artificial caput femur surface 45 is mounted in a functional position in the hip joint. A circular line 1519 is defining the major opening, which could further be defined as the smallest opening through which the caput femur can pass.

The area of the at least one first beyond part 1516 extending a distance D beyond the maximum diameter of the caput femur 5, is here adapted to hold a locking member in place. The locking member could be a cord or wire, and can be placed around the artificial caput femur surface 45 for further fixation of the medical device. The band, cord or wire can be mechanically connected using a self locking member for forming a ring-shaped element able to assist in the fixation of the artificial caput femur surface 45 to the caput femur 5.

The distance D from the circular line 1517 of the maximum outer distance on the artificial caput femur surface 45 to the circular line 1519 is defining the major opening of the medical device. The distance D is chosen to extend to the circular line 1519 which have a closest distance 1518 from the inner surface of the first beyond part 1518 to the medical device caput center axis 1504. When the at least one first beyond part 1516 is extending to the circular line 1519 the articulation or motion range of the hip joint is not limitied. According to the embodiment shown in fig. 6, the distance D is approximately 5 mm, however in other embodiments, the distance is approximately 10 mm.

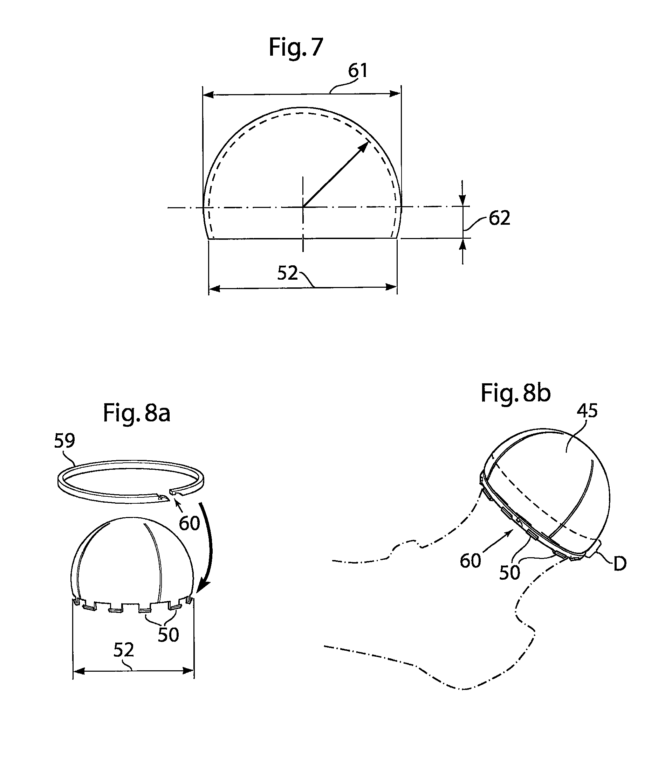

Fig. 7 shows an artificial caput femur surface 45 in section having a major opening 52 adapted to travel over and beyond the maximum diameter of the caput femur 5. The maximum diameter of the caput femur 5 is according to this embodiment positioned at a corresponding largest diameter 61 of the artificial caput femur surface. A second distance 62 is the distance that the artificial caput femur surface 45 travels beyond the maximum diameter of the caput femur 5. Said distance 62 is the beyond part of the artificial caput femur surface 45 and is a part that enabled the mechanical fixation of the artificial caput femur surface 45 to the caput femur 5, by said beyond part of the medical device exerting a squeezing force of the caput femur 5 and/or collum femur and clasps the caput femur 5.

Fig. 8a shows an artificial caput femur surface 45 according to one embodiment in which the artificial caput femur surface 45 is adapted to pass beyond the maximum diameter of the caput femur 5. This enables a mechanical fixation using the form of said artificial caput femur surface 45. In this embodiment the artificial caput femur surface 45 comprises at least one slit 49 adapted to make said artificial caput femur surface 45 flexible for traveling over and beyond the maximum diameter of the caput femur 5. It is also conceivable that the artificial caput femur surface 45 comprises two or more artificial caput femur surface arms 50 which create a largest diameter 52. This largest diameter 52 is according to one embodiment smaller the maximum diameter of the caput femur 5 enabling the mechanical fixation of the artificial caput femur surface 45 by means of said artificial caput femur surface arms 50. For further fixation, a locking member 59, which could be a cord or wire 59, can be placed around the artificial caput femur surface 45 beyond the maximum diameter of the caput femur 5. The band, cord or wire 59 can be mechanically connected using an engagementmember 60 for forming a loop-shaped element able to assist in the fixation of the artificial caput femur surface 45 to the caput femur 5.

Fig. 8b shows the artificial caput femur surface 45 when fixated to the caput femur 5 with the supporting band, cord or wire 59, as shown in fig. 8a, placed around the artificial caput femur surface 45 beyond the maximum diameter of the caput femur 5. The arms 50 may also be adapted to go into the bone of caput femur 5 to lock said artificial caput femur surface 45.

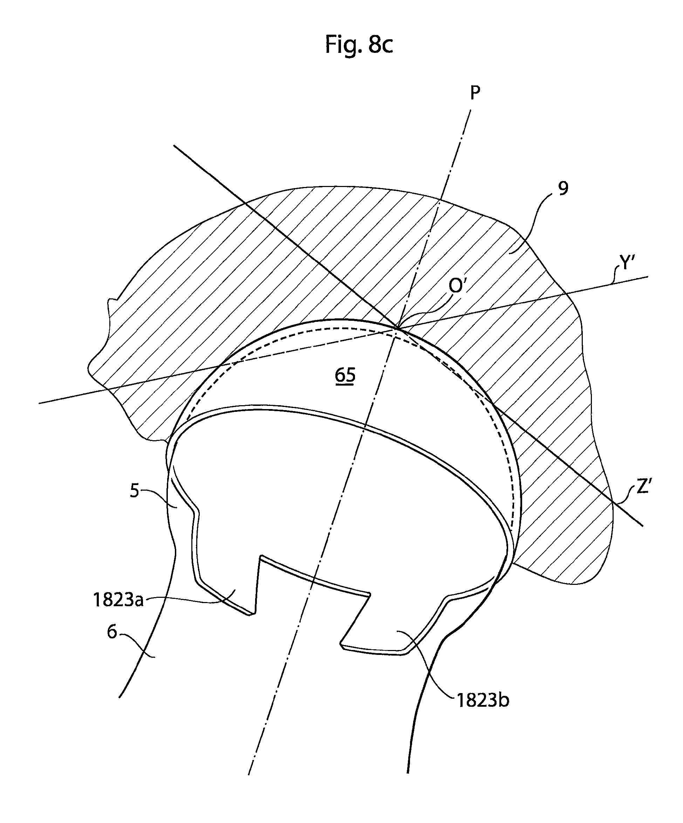

Fig. 8c shows the hip joint in section when a medical device has been implanted. The two extending portions 1823a and 1823b extending distally and clasping the spherical portion of the caput femur 5. The hip joint has a caput femur 5 integrated with a collum femur 6 having a center axis P extending longitudinal along the collum and caput femur in the center thereof.

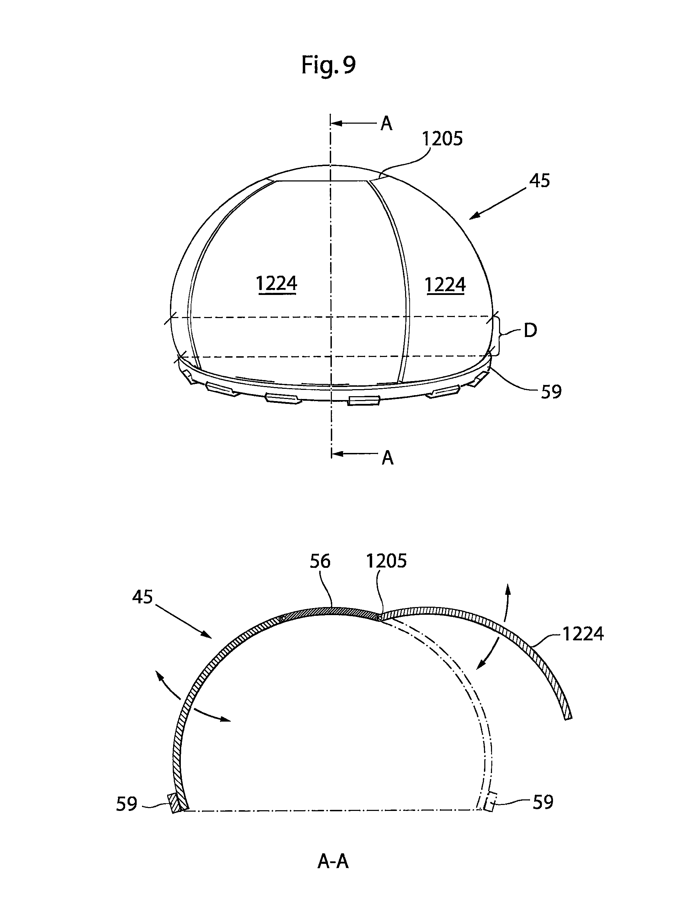

Fig. 9 shows an artificial caput femur surface 45 according to an embodiment in which the artificial caput femur surface comprises multiple movable portions 1224 connected to an interconnecting part 56 by operable joints 1205 placed along one side of the movable portions 1224. The artificial caput femur surface is further fixated to the caput femur by a band, cord or wire 59 placed beyond the maximum diameter of the caput femur 5 at a distance D from the maximum diameter and the most distal end of the moveable partitions 1224 directed away from the joints 1205. The artificial caput femur surface is fixated to the caput femur after the movable portions 1224 have been placed in there functional position clasping the caput femur 5, and be movable during an operation. The section A-A shows a movable portion 1224 when not in its functional state. The movable portion 1224 being connected to an interconnecting part 56 through a movable member in form of a hinge 1205 allowing the movable portion to move for being able to clasp the caput femur 5 and/or changing the maximum diameter of the artificial caput femur surface for passing through a hole smaller than the maximum diameter of the caput femur surface in its functional state, in which case the movable member is moved in a direction towards the center of the artificial caput femur surface (not shown).

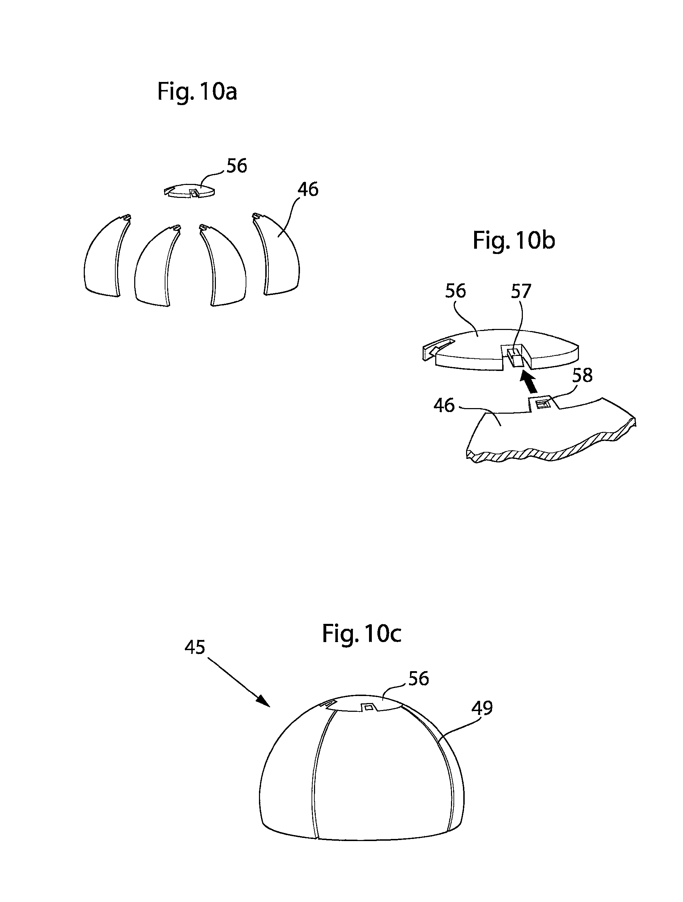

Fig. 10a-c shows an artificial caput femur surface 45 according to an embodiment in which the artificial caput femur surface comprises multiple portions 46 connected to an interconnecting part 56 by fastening means 57, 58 placed along one side of the portions 56. The fastening means comprises a first protruding portion 58 with an opening arranged on the one end of the portion 46. The opening in the first protruding portion 58 is adapted to interconnect with a second protruding portion 57 extending from a cut-out in the interconnection part 56. The multiple portions and the interconnection part can pass through a hole smaller than the maximum diameter of the caput femur surface and can be assembled to clasp the caput femur 5 after being inserted in the hip joint.

Fig. 11 a discloses the adjustable locking member 59 to be mounted on the artificial caput femur surface 45. The locking member 59 is a loop-shaped element having two ends 59a, 59b adapted to be mechanically connected using an engagement member 60, thus forming a closed loop with a certain circumference. The locking member 59 can be made out of an elastic material which deforms under stress (e.g. external forces), but returns to its original shape when the stress is removed.

The artificial caput femur surface 45 comprises two or more artificial caput femur surface arms 50 which create a largest diameter 52. To lock the artificial caput femur to the caput femur 5, the locking member 59 is, when it is in an open state, pulled over the surface 45 until it at least reaches an area extending a distance D beyond the maximum diameter of the caput femur 5. The locking member 59 can also be pulled until it reaches and rests on surface arms 50. When in its final position, the locking member ends 59a, 59b are mechanically connected by the engagement member 60, and the artificial caput femur is held in place.

Fig. 11b-11e shows different embodiments of the locking member 59 and the engagement member 60.

A first embodiment of a locking member 59 with engagement member 60 is disclosed in figure 11b. The engagement member 60 comprises a first and a second part 60a, 60b arranged in the first and second locking member end 59a, 59b, respectively. The first and second engagement member parts 60a, 60b have the shape of protrusions extending axially from the first and second locking member end, upwards and downward respectively. Thus forming a horizontally arranged gripping claw. The first engagement member part 60a has a cut-out in its lower surface and the second engagement member part 60b has a cut-out in its upper surface. The cut-outs are so arranged that they form an upper and a lower hook adapted to mechanically self connect by using the elasticity of the material and thus to form a loop with a certain circumference adapted to the diameter of the caput femur 5.

In a device according to the invention, including the locking member 59, shown in figure 11c, the engagement member 60' is arranged in one first and second end 59a, 59b of the locking member. In the first locking member end 59a one first engagement member part 60a' in the form of a protrusion extending radially, towards the center of the loop is arranged. The first engagement member part 60a is adapted to engage with one corresponding second engagement member part 60b which is a protrusion arranged in the other second end 59b of the locking member extending radially, from the center of the loop. The protrusions together are forming an engagement member in the form of a vertically arranged gripping claw 60'. The circumference of the locking member can be adjusted by using more than one second engagement member parts 60b and arranging them at different distances from the second end 59b of the locking member. In the second embodiment in figure 11c there are more than one, preferably between three and six, gripping claws 60b' arranged on the second end 59b of the locking member 59. The locking member 59 diameter can thus be adjusted.

Another locking member 59 in accordance with the invention is disclosed in figure 11d. In one first end 59a of the locking member 59 there is a first engagement member part 60a" in the form of a protrusion adapted to fit into a corresponding second engagement member part 60a" in the form of a recess or a hole in the other second end 59b of the locking member 59. In accordance with the invention, there is more than one hole so that the circumference of the locking member 59 is adjustable.

Another possible embodiment of the locking member 59 is disclosed in figure 11 e. Here the first and second ends 59a, 59b of the locking member 59 are connected by using an engagement member 60'" comprising two pivotable first locking parts 60a1"', 60a2'" and one second locking part 60b"'. The first locking part 60a1'" is pivotably attached both to the first end 59a of the locking member 59 and to the second locking part 60a2"'. The second locking part 60a2"' is attached to the first locking part 60a1'" in an engagement point arranged between the outer ends of the first locking part 60a1"', preferably in a point arranged substantially in the middle of the first locking part 60a1"'. The second locking part 60a2"' is also adapted to engage with a protruding part 60b"' arranged in the second end 59b of the locking member 59. When the second locking part 60a2'" is engaged with the protruding part 60b"', the first and second end of the locking member 59a, 59b is locked together forming a closed loop with a first circumference. The first and second locking member ends 59a, 59b can be pulled together forming a closed loop with a second circumference firmly enclosing the artificial caput femur and locking it to the caput femur 5. The first and second locking member ends 59a, 59b are pulled together by pivoting the first locking part around its engagement point in the first end 59a of the locking member. The first and second locking member ends 59a, 59b can be arranged either overlapping each other or being arranged end to end when locked together, thus forming a loop with the second circumference.

Fig. 12a shows yet another embodiment of the locking member, in which the locking member does not encircle the caput femur surface 45 completely, thus leaving a distance 2101 in which there is no locking member. According to the embodiment shown in fig. 12a the locking member 59 clamps the artificial caput femur surface by the locking member being made from an elastic material, such as stainless steel. The construction with locking member enables the artificial caput femur surface to be made from a more resilient material, for allowing the artificial caput femur surface to pass over the larger parts of the caput femur. One advantage with the embodiment shown in fig. 12 is that the locking member 59 does not have to be as elastic as the locking members that totally encircles the caput femur, to still be mountable by the surgeon in situ.

Fig. 12b shows a locking member according to an embodiment similar to the embodiment described previously, with reference to fig. 12a. However, according to the embodiment shown in fig. 12b, the locking member 59 comprises a hinge 2105 placed at the center of the locking member 59 to which two portions 2106a, 2106b of the locking member are connected. In connection to the hinge a locking device is placed comprising an male 2108 part adapted to connect to a female part 2109, thus creating a locking position. The locking member in the locking position clasps the artificial caput femur surface 45 and thus further fixates the artificial caput femur surface 45 to the caput femur. The embodiment of fig. 12b, with the hinge, enables the locking member to be made from a less elastic material than is necessary in embodiments where the entire locking member is made from a single piece of material (such as the embodiment described with reference to fig. 12a). The embodiment could further reduce the force needed to mount the locking member 59 onto the artificial caput femur 45 in situ.

Fig. 13a shows a locking member 59 according to yet another embodiment, in which the locking member 59 comprises a first and second unit 2102a, 2102b placed at two sides of a slit 49 in the artificial caput femur surface 45. The first unit 2102a comprises a male part 2103 which is insertable into a female part 2104 of the second unit 2102b, in which it locks and thus places the slit 49 in a more closed state for fixating the artificial caput femur 45 surface to the caput femur.

Fig. 13b shows the medical device according to an embodiment in which the locking member 59 is placed centrally in the top of an embodiment of the artificial caput femur surface 45, in which the artificial caput femur surface is dividable into two halves. The locking member comprises, in accordance with the embodiment shown with reference to fig. 13a, a first and second unit 2102a, 2102b, wherein said first unit comprises a male part 2103 adapted to lock inside of a female part housed in the second unit.

Fig 14 shows an embodiment of the locking member 59 in which the locking member 59 is adapted to travel from a first point of the artificial caput femur surface 45 through the bone of caput and/or collum femur and to a second point of the artificial caput femur surface 45. This embodiment could enable the locking member to fixate the artificial caput femur surface 45 to the caput femur by exerting a squeezing force and thus clamping the caput femur, and/or by the locking member 59 being inside the bone actually creating a mechanical lock thereby. According to the embodiment shown in fig. 14 the locking member 59 goes from one point of the artificial caput femur surface 45 to another point on the artificial caput femur surface 45, through the bone of the caput/collum femur. However in other embodiments (not shown) the locking member goes from a point of the artificial caput femur surface and into the bone of caput/collum femur, in these embodiments the locking members could be mechanical fixating members, such as orthopedic screws.

Fig. 15 shows a kit according to a first embodiment in which the kit comprises three different sizes of artificial caput femur surfaces 45a,b,c, which could be chosen on the basis of the particular patient, an a locking member 59 with several states which thus could be tightened around the different artificial caput femur surfaces 45a,b,c to fit the particular patient.

Fig. 16 shows a kit according to a second embodiment in which the kit comprises one artificial caput femur surface 45 and three different sizes locking member 59a,b,c which thus can be placed encircling the artificial caput femur surface 45 and be chosen for the particular femoral bone of a particular patient.

Fig. 17 shows a kit according to a second embodiment in which the kit comprises three different sizes of artificial caput femur surfaces 45a,b,c, which could be chosen on the basis of the particular patient, and three different sizes of locking members 59a,b,c which thus can be placed encircling the artificial caput femur surface 45 and be chosen for the particular femoral bone of a particular patient.

The kit solutions enables the orthopedic surgeon to choose a suitable medical device when the caput femur is exposed, since determining the exact size and shape of the caput femur is very hard from merely images created from outside of the body.

The medical device according to any of the embodiments could comprise at least one material selected from a group consisting of: polytetrafluoroethylene (PTFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/or carbon fibers and/or boron, a combination of metal and plastic materials, a combination of metal and carbon based material, a combination of carbon and plastic based material, a combination of flexible and stiff materials, a combination of elastic and less elastic materials, Corian or acrylic polymers.

## Claims

1. A medical device for implantation in a hip joint of a patient, the natural hip joint having a caput femur (5) integrated with a collum femur (6) having a center axis extending longitudinally along the collum and caput femur in the center thereof, the medical device comprising:
an artificial hollow caput femur surface (45), adapted to be fixated to at least one of the collum and caput femur, said artificial caput femur surface comprising at least one extending portion (1823) adapted to clasp a portion of caput and/or collum femur, when implanted, and
a locking member (59) adapted to assist in the fixation of the medical device, wherein said locking member comprises an element adapted to lock said artificial caput femur surface such that the at least one of the collum and caput femur remains clasped and restrained in said artificial hollow caput femur surface, **characterized in that** said locking member is adapted to lock in at least a first and a second predefined locking position.

2. The medical device according to claim 1, wherein said locking member is adapted to at least one of:
a. in said first locking position, lock a first artificial caput femur surface having at least one extending portion, to a caput femur and/or collum femur, and in said second locking position, lock a second smaller artificial caput femur surface having at least one extending portion, to the caput and/or collum femur,
b. in said first locking position, lock an artificial caput femur surface having at least one extending portion to a caput and/or collum femur, and in said second locking position, lock said artificial caput femur surface tighter to the caput and/or collum femur, and
c. in said first locking position, lock an artificial caput femur surface having at least one extending portion, to a first size caput and/or collum femur, and in said second locking position, lock said artificial caput femur surface, to a second smaller size caput femur and/or collum femur.

3. The medical device according to any one of the preceding claims, wherein said locking member comprises a loop-shaped element (59) adapted to be connected in situ for creating said loop shape surrounding the caput and/or collum femur, when implanted, and wherein said loop shaped element is adapted to have a first locking state, in which said loop shape has a first inner circumference, and wherein said loop shaped element is further adapted to have a second locking state, in which said loop shaped element has a second smaller inner circumference.

4. The medical device according to any one of the preceding claims, wherein said locking member is arranged in the extending portion of the artificial hollow caput femur surface, such that the locking member assists in the clasping of the at least one of the caput and collum femur.

5. The medical device according to any one of the preceding claims, wherein said locking member comprises a first and second engagement member (60), and wherein said first engagement member is adapted to engage said second engagement member, when implanted.

6. The medical device according to claim 5, wherein said first and second engagement members are adapted to mechanically self connect by introducing a male part of said first engagement member into a female part of said second engagement member.

7. The medical device according to claim 3, wherein said loop shaped element comprises a first and second end, and wherein said first and second ends are adapted to be connected to form said loop shaped element using an engagement member comprising a first and second pivotable locking part "60a1"'; 60a2'"".

8. The medical device according to claim 7, wherein said second locking part is attached to said first locking part in a point arranged substantially in the middle of said first locking part.

9. The medical device according to any one of the preceding claims, wherein said locking member is fixated to the artificial caput femur surface, when implanted, perpendicularly to said center axis of the caput and collum femur and partially surrounds the caput and/or collum femur and clasp the caput and/or collum femur, when implanted.

10. The medical device according to claim 9, wherein said locking member is made from an elastic material, for enabling the locking member to be placed onto the artificial caput femur surface, clasping the caput and/or collum femur.

11. The medical device according to any one of the preceding claims, wherein said locking member comprises at least one of:
a first and second portion, and wherein said first and second portions are pivotably connected at a hinge, and wherein said first and second portions are adapted to pivot for enabling the locking member to be placed partially surrounding the artificial caput femur, and
a locking element adapted to lock a connection between a first and a second portion, such that said locking member can be locked clasping the caput and/or collum femur, when implanted.

12. The medical device according to any one of the preceding claims, wherein said locking member comprises at least one mechanical fixating member (59) adapted to at least one of:
a. be placed in contact with the artificial caput femur surface, and thereby fixate the artificial caput femur surface to the bone of the collum and/or caput femur, and
b. travel from one point on the artificial caput femur surface, through a portion of the bone of the collum and/or caput femur, to a second point on the artificial caput femur surface, when implanted.

13. The medical device according to claim 13, wherein the length of the mechanical fixating member is changeable such that said mechanical fixating member can be shortened for squeezing and further fixating the artificial caput femur surface to the collum and/or caput femur.

14. The medical device according to any one of claims 12 and 13, wherein said mechanical fixating member is placed in contact with said artificial caput femur surface at the extending portion thereof.

15. The medical device according to any one of the preceding claims, wherein the extending portion of the artificial hollow caput femur surface is adapted to travel beyond the largest circumference of the caput femur, in the direction of the collum femur, when implanted.

## Patentansprüche

1. Medizinische Vorrichtung zur Implantierung in ein Hüftgelenk eines Patienten, wobei bei dem natürlichen Hüftgelenk der Caput femoris (5) in einem Collum femoris (6) integriert ist, bei dem sich eine Mittelachse in Längsrichtung entlang dem Collum und Caput femoris in dessen Mitte erstreckt, wobei die medizinische Vorrichtung folgendes umfasst:
eine Oberfläche (45) eines künstlichen hohlen Caput femoris, die angepasst ist, um an wenigstens eines aus Collum und Caput femoris angebracht zu werden, wobei die Oberfläche des künstlichen Caput femoris wenigstens einen sich erstreckenden Abschnitt (1823) umfasst, der angepasst ist, um, wenn implantiert, einen Abschnitt des Caput und/oder Collum femoris zu umfangen, und
ein Arretierungselement (59), das angepasst ist, um die Fixierung der medizinischen Vorrichtung zu unterstützen, wobei das Arretierungselement ein Element umfasst, dass angepasst ist, um die Oberfläche des künstlichen Caput femoris derart zu arretieren, dass wenigstens eines aus Collum und Caput femoris in der Oberfläche des künstlichen hohlen Caput femoris umfangen und gehalten werden, **dadurch gekennzeichnet, dass** das Arretierungselement angepasst ist, in wenigstens einer ersten und einer zweiten vorher definierten Arretierungsposition zu arretieren.

2. Medizinische Vorrichtung nach Anspruch 1, wobei das Arretierungselement angepasst ist, um wenigstens eines zu erfüllen, aus:
a. in der ersten Arretierungsposition, Arretieren einer Oberfläche des ersten künstlichen Caput femoris, die wenigstens einen sich erstreckenden Abschnitt aufweist, mit einem Caput femoris und/oder Collum femoris, und in der zweiten Arretierungsposition, Arretieren einer Oberfläche eines zweiten kleineren Caput femoris mit wenigstens einem sich erstreckenden Abschnitt, mit dem Caput femoris und/oder Collum femoris,
b. in der ersten Arretierungsposition, Arretieren einer Oberfläche des ersten künstlichen Caput femoris, die wenigstens einen sich erstreckenden Abschnitt aufweist, mit einem Caput femoris und/oder Collum femoris, und in der zweiten Arretierungsposition, festeres Arretieren der Oberfläche des künstlichen Caput femoris mit dem Caput und/oder Collum femoris, und
c. in der ersten Arretierungsposition, Arretieren einer Oberfläche des ersten künstlichen Caput femoris, die wenigstens einen sich erstreckenden Abschnitt aufweist, mit einem Caput femoris und/oder Collum femoris einer ersten Größe, und in der zweiten Arretierungsposition, Arretieren der Oberfläche des künstlichen Caput femoris mit einem Caput und/oder Collum femoris einer zweiten kleineren Größe.

3. Medizinische Vorrichtung nach einem der vorangehenden Ansprüche, wobei das Arretierungselement ein schleifenförmiges Element (59) umfasst, das angepasst ist, um in situ verbunden zu werden, um eine schleifenförmige Form zu bilden, die, wenn implantiert, den Caput und/oder Collum femoris umgibt, und wobei das schleifenförmige Element angepasst ist, um einen ersten Arretierungszustand zu haben, in welchem die Schleifenform einen ersten inneren Umfang aufweist, und wobei das schleifenförmige Element ferner angepasst ist, um einen zweiten Arretierungszustand aufzuweisen, in welchem das schleifenförmige Element einen zweiten kleineren inneren Umfang aufweist.

4. Medizinische Vorrichtung nach einem der vorangehenden Ansprüche, wobei das Arretierungselement in dem sich erstreckenden Abschnitt der Oberfläche des künstlichen hohlen Caput femoris angeordnet ist, derart, dass das Arretierungselement bei der Umfassung des wenigstens einen aus Caput und Collum femoris unterstützend wirkt.

5. Medizinische Vorrichtung nach einem der vorangehenden Ansprüche, wobei das Arretierungselement ein erstes und zweites Eingriffelement (60) umfasst, und wobei das erste Eingriffelement angepasst ist, um, wenn implantiert, das zweite Eingriffelement in Eingriff zu nehmen.

6. Medizinische Vorrichtung nach Anspruch 5, wobei die ersten und zweiten Eingriffelemente angepasst sind, sich mechanisch selbst zu verbinden, indem ein Steckerteil des ersten Eingriffelementes in ein Buchsenteil des zweiten Eingriffelementes eingeführt wird.

7. Medizinische Vorrichtung nach Anspruch 3, wobei das schleifenförmige Element ein erstes und zweites Ende umfasst, und wobei die ersten und zweiten Enden angepasst sind, um verbunden zu werden, um das schleifenförmige Element unter Verwendung eines Eingriffelementes, das ein erstes und zweites schwenkbares Verriegelungsteil "60a1'"; 60a2'"" umfasst, zu bilden.

8. Medizinische Vorrichtung nach Anspruch 7, wobei das zweite Arretierungsteil an dem ersten Arretierungsteil an einem Punkt angebracht wird, der im Wesentlichen in der Mitte des ersten Verriegelungsteils angeordnet ist.

9. Medizinische Vorrichtung nach einem der vorangehenden Ansprüche, wobei das Arretierungselement, wenn implantiert, an der Oberfläche des künstlichen Caput femoris fixiert wird, rechtwinklig zu der Mittelachse des Caput und Collum femoris und, wenn implantiert, den Caput und/oder Collum femoris teilweise umgibt und den Caput und/oder Collum femoris umgreift.

10. Medizinische Vorrichtung nach Anspruch 9, wobei das Arretierungselement aus einem elastischen Material hergestellt ist, damit das Arretierungselement auf der Oberfläche des künstlichen Caput femoris angeordnet werden kann und den Caput und/oder Collum femoris umgreift.

11. Medizinische Vorrichtung nach einem der vorangehenden Ansprüche, wobei das Arretierungselement wenigstens eines aus Folgendem umfasst:
einen ersten und zweiten Abschnitt, und wobei die ersten und zweiten Abschnitte schwenkbar an einem Scharnier befestigt sind, und wobei die ersten und zweiten Abschnitte angepasst sind, zu schwenken, damit das Arretierungselement so angeordnet werden kann, dass es den künstlichen Caput femoris teilweise umgibt, und
ein Arretierungselement, das angepasst ist, um eine Verbindung zwischen einem ersten und einem zweiten Abschnitt zu arretieren, derart, dass das Arretierungselement, wenn implantiert, derart arretiert werden kann, dass es den Caput und/oder Collum femoris umgreift.

12. Medizinische Vorrichtung nach einem der vorangehenden Ansprüche, wobei das Arretierungselement wenigstens ein mechanisches Fixierungselement (59) umfasst, das angepasst ist, um wenigstens eines aus:
a. in Kontakt mit der Oberfläche des künstlichen Caput femoris angeordnet zu sein und dabei die Oberfläche des künstlichen Caput femoris am Knochen des Collum und/oder Caput femoris zu fixieren, und
b. sich, wenn implantiert, von einem Punkt der Oberfläche des künstlichen Caput femoris durch einen Abschnitt des Knochens des Collum und/oder Caput femoris zu einem zweiten Punkt auf der Oberfläche des künstlichen Caput femoris zu bewegen.

13. Medizinische Vorrichtung nach Anspruch 13, wobei die Länge des mechanischen Fixierungselements änderbar ist, derart, dass das mechanische Fixierungselement zum Drücken und weiteren Fixieren der Oberfläche des künstlichen Caput femoris am Collum und/oder Caput femoris gekürzt werden kann.

14. Medizinische Vorrichtung nach einem der Ansprüche 12 und 13, wobei das mechanische Fixierungselement in Kontakt mit der Oberfläche des künstlichen Caput femoris an seinem sich erstreckenden Abschnitt angeordnet ist.

15. Medizinische Vorrichtung nach einem der vorangehenden Ansprüche, wobei der sich erstreckende Abschnitt der Oberfläche des künstlichen hohlen Caput femoris angepasst ist, um sich, wenn implantiert, über den größten Umfang des Caput femoris in Richtung des Collum femoris zu bewegen.

## Revendications

1. Dispositif médical destiné à être implanté dans une articulation de la hanche d'un patient, l'articulation de la hanche naturelle présentant une tête de fémur (5) intégrée à un col de fémur (6) dont l'axe central s'étend longitudinalement le long du col et de la tête du fémur, au centre de ces derniers, le dispositif médical comportant :
une surface (45) creuse artificielle pour tête de fémur, conçue pour être fixée sur au moins le col et/ou la tête du fémur, ladite surface artificielle pour tête de fémur comportant au moins une partie (1823) allongée conçue pour enserrer une partie de la tête et/ou du col du fémur, lorsqu'implantée, et
un élément (59) de verrouillage conçu pour faciliter la fixation du dispositif médical, ledit élément de verrouillage comportant un élément conçu pour verrouiller ladite surface artificielle pour tête de fémur de sorte qu'au moins le col et/ou la tête du fémur reste enserrée et maintenue dans ladite surface creuse artificielle pour tête de fémur, **caractérisé en ce que** ledit élément de verrouillage est conçu pour se verrouiller dans au moins une première et une seconde position de verrouillage prédéfinie.

2. Dispositif médical selon la revendication 1, ledit élément de verrouillage étant conçu pour au moins :
a. dans ladite première position de verrouillage, verrouiller une première surface artificielle pour tête de fémur présentent au moins une partie allongée, sur une tête de fémur et/ou un col de fémur, et dans ladite seconde position de verrouillage, verrouiller une seconde surface artificielle pour tête de fémur plus petite présentant au moins une partie allongée, sur la tête et/ou le col du fémur,
b. dans ladite première position de verrouillage, verrouiller une surface artificielle pour tête de fémur présentant au moins une partie allongée sur une tête et/ou un col du fémur, et dans ladite seconde position de verrouillage, verrouiller ladite surface artificielle pour tête de fémur de manière plus serrée sur la tête et/ou le col du fémur, et/ou
c. dans ladite première position de verrouillage, verrouiller une surface artificielle pour tête de fémur présentant au moins une partie allongée, sur une tête et/ou un col du fémur d'une première taille, et dans ladite seconde position de verrouillage, verrouiller ladite surface artificielle pour tête de fémur, sur une seconde tête de fémur et/ou col de fémur de taille plus petite.

3. Dispositif médical selon l'une quelconque des revendications précédentes, ledit élément de verrouillage comportant un élément (59) en forme de boucle conçu pour être relié *in situ* afin de créer ladite forme en boucle entourant la tête et/ou le col du fémur, lorsqu'implanté, et ledit élément en forme de boucle étant conçu pour avoir un premier état de verrouillage, dans lequel ladite forme en boucle a une première circonférence interne, et ledit élément en forme de boucle étant en outre conçu pour avoir un second état de verrouillage, dans lequel ledit élément en forme de boucle a une seconde circonférence interne plus petite.

4. Dispositif médical selon l'une quelconque des revendications précédentes, ledit élément de verrouillage étant agencé dans la partie allongée de la surface creuse artificielle pour tête de fémur, de sorte que l'élément de verrouillage facilite l'enserrement de ladite au moins une tête et/ou col du fémur.

5. Dispositif médical selon l'une quelconque des revendications précédentes, ledit élément de verrouillage comportant un premier et un second élément (60) de mise en prise, et ledit premier élément de mise en prise étant conçu pour mettre en prise ledit second élément de mise en prise, lorsqu'implanté.

6. Dispositif médical selon la revendication 5, lesdits premier et second éléments de mise en prise étant conçus pour se relier automatiquement mécaniquement en introduisant une partie mâle dudit premier élément de mise en prise dans une partie femelle dudit second élément de mise en prise.

7. Dispositif médical selon la revendication 3, ledit élément en forme de boucle comportant une première et une seconde extrémité, et lesdites première et seconde extrémités étant conçues pour être reliées afin de former ledit élément en forme de boucle en utilisant un élément de mise en prise comportant une première et une seconde partie "60a1"' ; 60a2'"" de verrouillage pouvant pivoter.

8. Dispositif médical selon la revendication 7, ladite seconde partie de verrouillage étant fixée à ladite première partie de verrouillage en un point agencé sensiblement au milieu de ladite première partie de verrouillage.

9. Dispositif médical selon l'une quelconque des revendications précédentes, ledit élément de verrouillage étant fixé à la surface artificielle pour tête de fémur, lorsqu'implanté, perpendiculairement audit axe central de la tête et du col du fémur et entourant partiellement la tête et/ou le col du fémur et enserrant la tête et/ou le col du fémur, lorsqu'implanté.

10. Dispositif médical selon la revendication 9, ledit élément de verrouillage étant fabriqué dans une matière élastique, pour permettre à l'élément de verrouillage d'être placé sur la surface artificielle pour tête de fémur, enserrant la tête et/ou le col du fémur.

11. Dispositif médical selon l'une quelconque des revendications précédentes, ledit élément de verrouillage comportant au moins :
une première et une seconde partie, et lesdites première et seconde parties étant reliées de manière pivotante au niveau d'une charnière, et lesdites première et seconde parties étant conçues pour pivoter afin de permettre à l'élément de verrouillage d'être placé de façon à enserrer partiellement la tête de fémur artificielle, et/ou
un élément de verrouillage conçu pour verrouiller une liaison entre une première et une seconde partie, de sorte que ledit élément de verrouillage peut être verrouillé enserrant la tête et/ou le col du fémur, lorsqu'implanté.

12. Dispositif médical selon l'une quelconque des revendications précédentes, ledit élément de verrouillage comportant au moins un élément (59) de fixation mécanique conçu pour au moins :
a. être placé en contact avec la surface artificielle pour tête de fémur et fixer ainsi la surface artificielle pour tête de fémur sur l'os du col et/ou de la tête du fémur, et
b. se déplacer depuis un point sur la surface artificielle pour tête de fémur, à travers une partie de l'os du col et/ou de la tête du fémur, jusqu'à un second point sur la surface artificielle pour tête de fémur, lorsqu'implanté.

13. Dispositif médical selon la revendication 13, la longueur de l'élément de fixation mécanique pouvant être changée de sorte que ledit élément de fixation mécanique peut être raccourci pour appuyer et fixer davantage la surface surface artificielle pour tête de fémur sur le col et/ou la tête de fémur.

14. Dispositif médical selon l'une quelconque des revendications 12 et 13, ledit élément de fixation mécanique étant placé en contact avec ladite surface artificielle pour tête de fémur au niveau de sa partie allongée.

15. Dispositif médical selon l'une quelconque des revendications précédentes, la partie allongée de la surface creuse artificielle pour tête de fémur étant conçue pour se déplacer au-delà de la plus grande circonférence de la tête du fémur, dans la direction du col du fémur, lorsqu'implantée.
